# EUROPEAN PATENT APPLICATION

(11) **EP 3 552 598 A1**
(43) Date of publication of application: **16.10.2019**
(21) Application number: 19168759.9
(22) Date of filing: 11.04.2019
(51) Int. Cl.: A61K 8/73, A61Q 19/00, A61K 8/9711, A61K 8/9783

(54) **COMPOSITION CONTAINING CROCUS SATIVUS EXTRACT AND PREPARATION METHOD AND USE THEREOF**

(30) Priority: 12.04.2018 CN 201810326375
(71) Applicant: Shanghai Traditional Chinese Medicine Co., Ltd., Shanghai 200002 (CN)
(72) Inventor: ZHANG, Xue, Shanghai, Shanghai 200002 (CN); LIU, Guodong, Shanghai, Shanghai 200002 (CN); SI, Ruirong, Shanghai, Shanghai 200002 (CN)
(74) Representative: Martegani, Franco

(57) **Abstract**

The present invention provides a composition containing *Crocus sativus* extract and a preparation method and use thereof. The composition containing *Crocus sativus* extract comprises the following raw materials in parts by mass: 4-6 parts of sodium hyaluronate, 0.5-1.5 parts of *Crocus sativus* extract, 0.5-1.5 parts of *Cladosiphon okamuranus* extract and 0.5-1.5 parts of *Chrysanthellum indicum* extract. The composition containing *Crocus sativus* extract prepared by the present invention has a long-lasting effect of resisting oxidation and moisturizing the skin. The " *Crocus sativus* Brightening Silk Mask" prepared with the composition containing *Crocus sativus* extract of the present invention has a good effect of brightening complexion and resisting oxidation, and the " *Crocus sativus* Eye-Brightening & Soothing & Tightening Eye Mask" prepared has a good anti-wrinkle effect.

## Description

### FIELD OF THE INVENTION

The present invention relates to a composition containing *Crocus sativus* extract and a preparation method and use thereof.

### BACKGROUND OF THE INVENTION

*Crocus sativus* L is one of the raw materials for both medicine and food approved by China's Ministry of Health. Its main medicinal ingredients includes: crocin I and crocin II, no less than 10%; crocin, about 1.5%; crocetin dimethyl ester; picrocrocin, about 2%; and volatile oil, 0.4% to 1.3%. In addition, it also contains compounds such as eucalyptol, pinene, daucosterol, and multivitamins, etc.

*Crocus sativus* L is still widely used in medical fields at home and abroad, and is further widely recognized as a good product for invigorating the circulation of blood and beautifying the skin. Its application mainly includes the following:
Cleaning: The theory of free radical damage suggests that the accumulation of oxygen free radicals produced by aerobic metabolism damages various biological macromolecules and eventually leads to aging. Some antioxidants such as vitamin E, vitamin C, and carotene, etc. can scavenge the free radicals. A facial cleanser containing *Crocus sativus* extract may thoroughly remove dirt in the pores and improve the anti-oxidation and anti-aging abilities of the skin.
Repair: Modern woman bears tremendous pressure from work and life, which may easily cause anxiety, reduced sleep quality, increased blood viscosity, and blockage of capillaries that are invisible to the naked eye. Clinical studies have shown that the hot water extract of *Crocus sativus* has obvious inhibitory effect on blood coagulation, and adenosine, as its active ingredient, can prolong the generation time and activation time of prothrombin and lower the whole blood specific viscosity. A hydrating moisturizing cream containing *Crocus sativus* extract can replenish moisture for skin while shrinking the pores, and in addition, it can further exert its functions of invigorating the circulation of blood and resisting hypoxia, and significantly improve blood microcirculation.
Food: The beautiful appearance of *Crocus sativus* helps increase the color and flavor of cooked foods. Due to carotene, crocin, cis-crocetin dimethyl ester and other active ingredients contained in its stigma, if a stigma of *Crocus sativus* is placed in a glass of water, the full glass of water presents a beautiful golden yellow color, which gives a person with very good visual aesthetic sense while beautifying the skin.

However, when the *Crocus sativus* extract is used alone, it is difficult to maintain its effect of brightening complexion and resisting oxidation for a long time. Therefore, it has great commercial value to develop a facial mask containing *Crocus sativus* extract which can maintain the effect of brightening complexion and resisting oxidation for a long time, and it is also an urgent problem to be solved in the art.

### SUMMARY OF THE INVENTION

In order to overcome the defects in the prior art that it is difficult to maintain the effect of the *Crocus sativus* extract for brightening complexion and moisturizing the skin, the present invention provides a composition containing *Crocus sativus* extract and a preparation method and use thereof. The facial mask prepared with the composition containing *Crocus sativus* extract provided by the present invention has a long-lasting effect of brightening complexion and resisting oxidation.

The present invention solves the above technical problems by the following technical solutions.

The present invention provides a composition containing *Crocus sativus* extract, which comprises the following raw materials in parts by mass: 4-6 parts of sodium hyaluronate, 0.5-1.5 parts of *Crocus sativus* extract, 0.5-1.5 parts of *Cladosiphon okamuranus* extract and 0.5-1.5 parts of *Chrysanthellum indicum* extract.

In the present invention, the sodium hyaluronate may be a conventional sodium hyaluronate in the art, preferably purchased from Bloomage Freda Biopharm Co., Ltd.

In the present invention, the *Crocus sativus* extract may be obtained by a conventional method in the art, and is generally obtained by the following method: mixing *Crocus sativus* L with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Crocus sativus* L is produced from a professional planting base for *Crocus sativus* L of Shanghai traditional Chinese medicine Co., Ltd. in Chongming Island.

In the present invention, the *Cladosiphon okamuranus* extract may be obtained by a conventional method in the art, and is generally obtained by the following method: mixing *Cladosiphon okamuranus* with a solvent and extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Cladosiphon okamuranus* extract is purchased from Liaoyuan Daily Chemical Co., Ltd.

In the present invention, the *Chrysanthellum indicum* extract may be obtained by a conventional method in the art, and is generally obtained by the following method: mixing *Chrysanthellum indicum* with a solvent and extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Chrysanthellum indicum* extract is purchased from Liaoyuan Daily Chemical Co., Ltd.

In the present invention, the content of the sodium hyaluronate is preferably 5 parts.

In the present invention, the content of the *Crocus sativus* extract is preferably 1 part.

In the present invention, the content of the *Cladosiphon okamuranus* extract is preferably 1 part.

In the present invention, the content of the *Chrysanthellum indicum* extract is preferably 1 part.

In the present invention, the composition may further comprise one or more of a solvent, a humectant, a thickener, a pH adjuster, a solubilizer, an antioxidant, a skin conditioner, a preservative, and a fragrance.

Wherein, the solvent may be a conventional one in the art, such as water.

Wherein, the humectant may be a conventional one in the art, such as propylene glycol.

Wherein, the content of the humectant may be a conventional content in the art, for example, the mass ratio of the humectant to the *Crocus sativus* extract is preferably (1400 to 1600): 1, and more preferably 1500:1.

Wherein, the thickener may be a conventional one in the art, such as hydroxyethyl cellulose.

Wherein, the content of the thickener may be a conventional content in the art, for example, the mass ratio of the thickener to the *Crocus sativus* extract is preferably (30 to 50): 1, and more preferably 40:1.

Wherein, the pH adjuster may be a conventional one in the art, such as triethanolamine.

Wherein, the content of the pH adjuster may be a conventional content in the art, which is a content of the pH adjuster required to adjust to a suitable pH range according to the common knowledge in the art.

Wherein, the solubilizer may be a conventional one in the art, such as PEG-40 hydrogenated castor oil.

Wherein, the content of the solubilizer may be a conventional content in the art, for example, the mass ratio of the solubilizer to the *Crocus sativus* extract is preferably (4 to 6):1, and more preferably 5:1.

Wherein, the antioxidant may be a conventional one in the art, such as tocopheryl acetate and/or palmitoyl pentapeptide-4.

Wherein, the content of the antioxidant may be a conventional content in the art, for example, the mass ratio of the antioxidant to the *Crocus sativus* extract is preferably (0.5 to 1.5): 1, and more preferably 1:1.

Wherein, the skin conditioner may be a conventional one in the art, such as one or more of dipotassium glycyrrhizinate, *Nymphaea odorata* root extract, hydrolyzed silk and *Camellia sinensis* catechins, and preferably "dipotassium glycyrrhizinate, *Nymphaea odorata* root extract and hydrolyzed silk" or "dipotassium glycyrrhizinate and *Camellia sinensis* catechins".

The *Nymphaea odorata* root extract may be obtained by a conventional method in the art, and is generally obtained by the following method: mixing *Nymphaea odorata* root with a solvent and extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Nymphaea odorata* root extract is purchased from Liaoyuan Daily Chemical Co., Ltd.

The *Camellia sinensis* catechins are generally phenolic active substances extracted from *Camellia sinensis;* and preferably, the *Camellia sinensis* catechins are purchased from Liaoyuan Daily Chemical Co., Ltd. The *Camellia sinensis* catechins are described in International Nomenclature Cosmetic Ingredient (INCI).

The hydrolyzed silk may be obtained by a conventional method in the art, and is generally obtained by hydrolyzing a cocoon shell to fibroin. Preferably, the hydrolyzed silk is purchased from Liaoyuan Daily Chemical Co., Ltd.

Wherein, the content of the skin conditioner is a conventional content in the art, and the mass ratio of the skin conditioner to the *Crocus sativus* extract is preferably (1 to 12): 1, for example, 12:1, or 2:1.

When the skin conditioner is dipotassium glycyrrhizinate, *Nymphaea odorata* root extract and hydrolyzed silk, the mass ratio of the dipotassium glycyrrhizinate, the *Nymphaea odorata* root extract to the hydrolyzed silk is preferably 10:1:1.

When the skin conditioner is dipotassium glycyrrhizinate and *Camellia sinensis* catechins, the mass ratio of the dipotassium glycyrrhizinate to the *Camellia sinensis* catechins is preferably 1:1.

Wherein, the preservative can be a conventional one in the art, such as chlorphenesin.

Wherein, the content of the preservative may be a conventional content in the art, for example, the mass ratio of the preservative to the *Crocus sativus* extract is preferably (15 to 25):1, and more preferably 20:1.

Wherein, the content of the fragrance may be a conventional content in the art, for example, the mass ratio of the fragrance to the *Crocus sativus* extract is preferably (0.5 to 1.5): 1, and more preferably 1:1.

In a preferred embodiment of the present invention, the composition containing *Crocus sativus* extract comprises the following raw materials in parts by weight: 5 parts of sodium hyaluronate, 1 part of *Crocus sativus* extract, 1 part of *Cladosiphon okamuranus* extract and 1 part of *Chrysanthellum indicum* extract.

In a preferred embodiment of the present invention, the composition containing *Crocus sativus* extract comprises, 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Nymphaea odorata* root extract, 0.01% *Cladosiphon okamuranus* extract, 0.01% *Chrysanthellum indicum* extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

In a preferred embodiment of the present invention, the composition containing *Crocus sativus* extract comprises, 84.23% water, 15% propylene glycol, 0.4% hydroxyethyl cellulose, 0.05% PEG-40 hydrogenated castor oil, 0.01% palmitoyl pentapeptide-4, 0.05% sodium hyaluronate, 0.01% *Crocus sativus* extract, 0.01% *Camellia sinensis* catechins, 0.01% *Cladosiphon okamuranus* extract, 0.01% *Chrysanthellum indicum* extract, 0.01% dipotassium glycyrrhizinate, 0.2% chlorphenesin and 0.01% fragrance, and the above percentages refer to the mass percentage accounting for the total amount of the raw materials.

The present invention also provides a preparation method of the above composition containing *Crocus sativus* extract, wherein the raw material components of the composition containing *Crocus sativus* extract are mixed.

When the raw materials contain other component(s) in addition to the sodium hyaluronate, the *Crocus sativus* extract, the *Cladosiphon okamuranus* extract and the *Chrysanthellum indicum* extract, the other component(s) is/are firstly mixed, and then mixed with "the sodium hyaluronate, the *Crocus sativus* extract, the *Cladosiphon okamuranus* extract and the *Chrysanthellum indicum* extract".

The present invention also provides use of the above composition containing *Crocus sativus* extract in cosmetics.

In the present invention, the cosmetic may be a conventional cosmetic in the art, such as a facial mask or an eye mask.

Wherein, the raw material of the facial mask preferably includes: a solvent, a humectant, a thickener, a pH adjuster, a solubilizer, an antioxidant, sodium hyaluronate, *Crocus sativus* extract, *Cladosiphon okamuranus* extract, *Chrysanthellum indicum* extract, *Nymphaea odorata* root extract, hydrolyzed silk, dipotassium glycyrrhizinate, a preservative and a fragrance.

The definition of the solvent, the humectant, the thickener, the pH adjuster, the solubilizer, the antioxidant, the sodium hyaluronate, the *Crocus sativus* extract, the *Cladosiphon okamuranus* extract, the *Chrysanthellum indicum* extract, the *Nymphaea odorata* root extract, the hydrolyzed silk, the dipotassium glycyrrhizinate, the preservative and the fragrance are as described above.

The raw materials of the facial mask are preferably the following components: 84.12% water, 15% propylene glycol, 0.4% hydroxyethyl cellulose, 0.01% triethanolamine, 0.05% PEG-40 hydrogenated castor oil, 0.05% sodium hyaluronate, 0.01% tocopheryl acetate, 0.01% *Crocus sativus* extract, 0.01% *Nymphaea odorata* root extract, 0.01% *Cladosiphon okamuranus* extract, 0.01% *Chrysanthellum indicum* extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

The preparation method of the facial mask includes the following steps:
(1) mixing a group A and a group B, and homogenizing to obtain a mixture 1; the group A includes the humectant, and the group B includes the thickener, the pH adjuster, the sodium hyaluronate, the dipotassium glycyrrhizinate, the hydrolyzed silk and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Crocus sativus* extract, the *Cladosiphon okamuranus* extract, the *Chrysanthellum indicum* extract and the *Nymphaea odorata* root extract.

In the step (1), the method for mixing the group A and the group B may be a conventional method in the art, for example, mixing the raw materials; preferably, separately adding the raw materials of the group B to the group A; and more preferably, separately adding the raw materials of the group B to the group A after homogenization. The time for homogenizing may be a conventional one in the art, for example, 20 min.

In the step (1), the time for homogenizing is preferably 25 min.

In the step (1), preferably, the mixture 1 is filtered. The mesh number of the filter screen for filtration may be a conventional one in the art, for example, 300 mesh.

In the step (2), the time for homogenizing is preferably 60 to 90 min.

In the step (3), the preparation method of the group C may be a conventional one in the art, for example, uniformly stirring the solubilizer, the antioxidant and the fragrance. The time for stirring may be a conventional one in the art, for example, 10 min.

In the step (3), the time for homogenizing is preferably 25 min.

In the step (4), preferably, the *Crocus sativus* extract, the *Cladosiphon okamuranus* extract, the *Chrysanthellum indicum* extract and the *Nymphaea odorata* root extract are separately added to the mixture 3.

In the step (4), the time for homogenizing is preferably 20 min.

Wherein, the raw material of the eye mask preferably includes: a solvent, a humectant, a thickener, a solubilizer, an antioxidant, sodium hyaluronate, *Crocus sativus* extract, *Cladosiphon okamuranus* extract, *Chrysanthellum indicum* extract, *Camellia sinensis* catechins, dipotassium glycyrrhizinate, a preservative and a fragrance.

The definition of the solvent, the humectant, the thickener, the solubilizer, the antioxidant, the sodium hyaluronate, the *Crocus sativus* extract, the *Cladosiphon okamuranus* extract, the *Chrysanthellum indicum* extract, the *Nymphaea odorata* root extract, the *Camellia sinensis* catechins, the dipotassium glycyrrhizinate, the preservative and the fragrance are as defined above.

The raw materials of the eye mask are preferably the following components: 84.23% water, 15% propylene glycol, 0.4% hydroxyethyl cellulose, 0.05% PEG-40 hydrogenated castor oil, 0.05% sodium hyaluronate, 0.01% palmitoyl pentapeptide-4, 0.01% *Crocus sativus* extract, 0.01% *Chrysanthellum indicum* extract, 0.01% *Camellia sinensis* catechins, 0.01% *Cladosiphon okamuranus* extract, 0.01% dipotassium glycyrrhizinate, 0.2% chlorphenesin and 0.01% fragrance, and the above percentages refer to the mass percentage accounting for the total amount of the raw materials.

The preparation method of the eye mask includes the following steps:
(1) mixing a group A and a group B, and homogenizing to obtain a mixture 1; the group A includes the humectant, and the group B includes the thickener, the sodium hyaluronate, the dipotassium glycyrrhizinate and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Crocus sativus* extract, the *Cladosiphon okamuranus* extract, the *Chrysanthellum indicum* extract and the *Camellia sinensis* catechins.

In the step (1), the mixing method of the group A and the group B may be a method conventional in the art, for example, mixing the raw materials; preferably, separately adding the raw materials of the group B to the group A; and more preferably, separately adding the raw materials of the group B to the group A after homogenization. The time for homogenizing may be a conventional one in the art, for example, 20 min.

In the step (1), the time for homogenizing is preferably 25 min.

In the step (1), preferably, the mixture 1 is filtered. The mesh number of the filter screen for filtration may be a conventional one in the art, for example, 300 mesh.

In the step (2), the time for homogenizing is preferably 60 to 90 min.

In the step (3), the preparation method of the group C may be a conventional one in the art, for example, uniformly stirring the solubilizer, the antioxidant, and the fragrance. The time for stirring may be a conventional one in the art, for example, 10 min.

In the step (3), the time for homogenizing is preferably 25 min.

In the step (4), preferably, the *Crocus sativus* extract, the *Cladosiphon okamuranus* extract, the *Chrysanthellum indicum* extract and the *Camellia sinensis* catechins are separately added to the mixture 3.

In the step (4), the time for homogenizing is preferably 20 min.

Based on the common knowledge in the art, the above various preferred conditions may be arbitrarily combined to obtain preferred embodiments of the present invention.

The reagents and raw materials used in the present invention are commercially available.

The beneficial effects of the present invention are that:
(1) The composition containing *Crocus sativus* extract prepared by the present invention has a long-lasting effect of resisting oxidation and brightening complexion.
(2) The *"Crocus sativus* Brightening Silk Facial Mask" prepared with the composition containing *Crocus sativus* extract of the present invention has a good effect of brightening complexion, and after applying the facial mask continuously for four weeks and then suspending use for three weeks, the skin may still remain as bright as it was after two weeks of use.
(3) The *"Crocus sativus* Eye-Brightening & Soothing & Tightening Eye Mask" prepared with the composition containing *Crocus sativus* extract of the present invention has a good anti-wrinkle effect. 20 volunteers performed sensory evaluation on it, and the ratio of 5 points (out of 5 points) reached 80%.
(4) The preparation process adopted in the present application is simple and suitable for industrial production.

### DETAILED DESCRIPTION OF THE INVENTION

The invention is further illustrated by the following examples, which are not intended to limit the invention. The experimental methods in the following examples, which do not specify the specific conditions, are selected according to conventional methods and conditions, or according to the manufacturer's instructions.

In the following examples, sodium hyaluronate was purchased from Bloomage Freda Biopharm Co., Ltd.

In the following examples, *Cladosiphon okamuranus* extract, *Chrysanthellum indicum* extract, *Nymphaea odorata* root extract and hydrolyzed silk were purchased from Liaoyuan Daily Chemical Co., Ltd.

In the following examples, the preparation method of the *Crocus sativus* extract was: using *Crocus sativus* L produced from the professional planting base for *Crocus sativus* L of Shanghai traditional Chinese medicine Co., Ltd. in Chongming Island, and extracting it according to a conventional method in the art. The extraction solvent may be water and/or ethanol. The extraction method may be a dipping method, a percolating method, a decocting method, a refluxing method or a continuous extraction method, etc.

### Example 1

In this example, the formula of the composition containing *Crocus sativus* extract and the parts by mass of each component were: sodium hyaluronate, 5 parts; *Crocus sativus* extract, 1 part; *Cladosiphon okamuranus* extract, 1 part; and *Chrysanthellum indicum* extract, 1 part.

The composition was prepared by mixing sodium hyaluronate, *Crocus sativus* extract, *Cladosiphon okamuranus* extract and *Chrysanthellum indicum* extract according to their mass ratios.

### Example 2

In this example, the formula of the composition containing *Crocus sativus* extract and the parts by mass of each component were: sodium hyaluronate, 5 parts; *Crocus sativus* extract, 1 part; *Cladosiphon okamuranus* extract, 1 part; *Chrysanthellum indicum* extract, 1 part; *Nymphaea odorata* root extract, 1 part; dipotassium glycyrrhizinate, 10 parts; and hydrolyzed silk, 1 part.

The composition was prepared by mixing sodium hyaluronate, *Crocus sativus* extract, *Cladosiphon okamuranus* extract, *Chrysanthellum indicum* extract, *Nymphaea odorata* root extract, dipotassium glycyrrhizinate and hydrolyzed silk according to their mass ratios.

### Example 3

In this example, the formula of the composition containing *Crocus sativus* extract and the parts by mass of each component were: sodium hyaluronate, 5 parts; *Crocus sativus* extract, 1 part; *Cladosiphon okamuranus* extract, 1 part; *Chrysanthellum indicum* extract, 1 part; *Camellia sinensis* catechins, 1 part; and dipotassium glycyrrhizinate, 1 part.

The composition was prepared by mixing sodium hyaluronate, *Crocus sativus* extract, *Cladosiphon okamuranus* extract, *Chrysanthellum indicum* extract, *Camellia sinensis* catechins and dipotassium glycyrrhizinate according to their mass ratios.

### Example 4

The raw material formula of the facial mask was: water 84.12%, propylene glycol 15%, hydroxyethyl cellulose 0.4%, triethanolamine 0.01%, PEG-40 hydrogenated castor oil 0.05%, sodium hyaluronate 0.05%, tocopheryl acetate 0.01%, *Crocus sativus* extract 0.01%, *Nymphaea odorata* root extract 0.01%, *Cladosiphon okamuranus* extract 0.01%, *Chrysanthellum indicum* extract 0.01%, hydrolyzed silk 0.01%, dipotassium glycyrrhizinate 0.1%, chlorphenesin 0.2% and fragrance 0.01%, and the above percentages refer to the mass percentage accounting for the total amount of the raw materials.

Preparation method of the facial mask: according to the above mass percentage, propylene glycol was homogenized in a homogenizer for 25 min, then hydroxyethyl cellulose, triethanolamine, sodium hyaluronate, hydrolyzed silk, dipotassium glycyrrhizinate and chlorphenesin were added, and the resulting mixture was homogenized for 25 min, and then water was added, and the resulting mixture was homogenized for 90 min, then PEG-40 hydrogenated castor oil, tocopherol acetate and fragrance were added, and the resulting mixture was homogenized for 25 min, finally, *Crocus sativus* extract, *Nymphaea odorata* root extract, *Cladosiphon okamuranus* extract and *Chrysanthellum indicum* extract were added, and the homogenized mixture was filtered through a 300 mesh filter screen.

### Example 5

The raw material formula of the eye mask was: water 84.23%, propylene glycol 15%, hydroxyethyl cellulose 0.4%, PEG-40 hydrogenated castor oil 0.05%, sodium hyaluronate 0.05%, palmitoyl pentapeptide-4 0.01%, *Crocus sativus* extract 0.01%, *Chrysanthellum indicum* extract 0.01%, *Camellia sinensis* catechins 0.01%, *Cladosiphon okamuranus* extract 0.01%, dipotassium glycyrrhizinate 0.01%, chlorphenesin 0.2% and fragrance 0.01%, and the above percentages refer to the mass percentage accounting for the total amount of the raw materials.

Preparation method of the eye mask: according to the above mass percentage, propylene glycol was homogenized in a homogenizer for 25 min, then hydroxyethyl cellulose, sodium hyaluronate, dipotassium glycyrrhizinate and chlorphenesin were added, and the resulting mixture was homogenized for 25 min, and then water was added, and the resulting mixture was homogenized for 90 min, and PEG-40 hydrogenated castor oil, palmitoyl pentapeptide-4 and fragrance were then added, the resulting mixture was homogenized for 25 min, finally, *Crocus sativus* extract, *Chrysanthellum indicum* extract, *Camellia sinensis* catechins and *Cladosiphon okamuranus* extract were added, and the homogenized mixture was filtered through a 300 mesh filter screen.

### Effect Example 1

According to the SOD oxidase detection experiment, the composition prepared in Example 1 had good antioxidant activity, and had a more durable antioxidant effect than the *Crocus sativus* extract alone.

### Effect Example 2

The facial mask prepared in Example 4 may be used in the peel-off mask or may be directly applied to the face.

### (1) Instant use effect of the facial mask

Twenty volunteers aged between 30 and 40 years old were selected. After facial cleansing, they used the facial mask prepared in Example 4 of this application. The scores after use are as follows.

**Table 1**

| | |
|---|---|
| 5 points | The skin is moist, tender and smooth, plump and elastic, with a brightened complexion, and the skin is soft and silky. |
| 4 points | The skin is smooth, with a brightened complexion. |
| 3 points | The skin is smooth. |
| 2 points or less | No significant improvement on skin is found |

**Table 2**

| | |
|---|---|
| 5 points | 16 |
| 4 points | 3 |
| 3 points | 1 |
| 2 points or less | 0 |

It can be seen from Table 1 and Table 2 that the facial mask prepared by the Example 4 of this application had a good effect of brightening complexion and resisting oxidation, and the score rate of 5 points reached 80%.

### (2) Long-term use effect of the facial mask

Twenty women aged between 30 and 40 were selected, who separately used the facial mask product prepared in Example 4 and a certain brand of Brightening & Whitening cosmetic purchased on the market (as Comparative Example 1). The skin brightness was measured using a skin melanin and hemoglobin tester (Mexameter 18, CK, Germany). The tester has a measurement range of 0 to 999. The higher the measurement value, the higher the content of melanin and hemoglobin in the skin.

The facial mask was applied once a day for the first three days, and then once every two days, for four weeks. Before use, the skin brightness was measured under controlled conditions of a constant temperature of 25°C and a relative humidity of 40% as a reference. The skin brightness was measured after one week, two weeks, and four weeks of use, and three weeks after the suspension of use. The average skin brightness is shown in the table below.

**Table 3**

| | Before use | One week | Two weeks | Four weeks | After three weeks of suspension |
|---|---|---|---|---|---|
| Example 3 | 660 | 553 | 501 | 460 | 498 |
| Comparative Example 1 | 656 | 575 | 563 | 544 | 623 |

As can be seen from Table 3, based on the values measured by the skin melanin and hemoglobin tester before the experiment, the skin brightness was significantly improved after a predetermined period of use. Compared with the certain brand of Brightening & Whitening cosmetic, the facial mask prepared by this application had better performance for brightening complexion. Moreover, even after three weeks of suspension, the skin still remained as bright as it was after two weeks of use, indicating that the facial mask prepared in Example 4 has a long-lasting effect of brightening complexion and whitening the skin.

The facial mask prepared by the composition formulae of Example 1 and Example 2 had an effect comparable to that of Example 4.

### Effect Example 3

The eye mask prepared in Example 5 may be directly applied to the eye. Twenty female volunteers aged between 30 and 40 were selected. After cleansing, they used the eye mask prepared in Example 5 of this application. The scores after use are as follows.

**Table 4**

| | |
|---|---|
| 5 points | The skin around the eyes is smooth, the fine lines are smoothed, the dryness of the eye skin is significantly improved, and the complexion is brightened. |
| 4 points | The skin around the eyes is smooth, and the complexion is brightened. |
| 3 points | The skin around the eyes is smooth. |
| 2 points or less | No significant improvement on skin around the eyes is found. |

**Table 5**

| | |
|---|---|
| 5 points | 17 |
| 4 points | 2 |
| 3 points | 1 |
| 2 points or less | 0 |

It can be seen from Table 4 and Table 5 that the eye mask prepared in Example 3 of this application had a good effect of resisting wrinkle and brightening complexion, and the score rate of 5 points reached 85%.

The eye mask prepared by the composition formulae of Example 1 and Example 3 had an effect comparable to that of Example 5.

## Claims

1. A composition containing *Crocus sativus* extract, comprising the following raw materials in parts by mass: 4-6 parts of sodium hyaluronate, 0.5-1.5 parts of *Crocus sativus* extract, 0.5-1.5 parts of *Cladosiphon okamuranus* extract and 0.5-1.5 parts of *Chrysanthellum indicum* extract.

2. The composition of claim 1, wherein the content of the sodium hyaluronate is 5 parts,
and/or, the content of the *Crocus sativus* extract is 1 part,
and/or, the content of the *Cladosiphon okamuranus* extract is 1 part,
and/or, the content of the *Chrysanthellum indicum* extract is 1 part.

3. The composition of claim 1 or 2, wherein the composition further comprises one or more of a solvent, a humectant, a thickener, a pH adjuster, a solubilizer, an antioxidant, a skin conditioner, a preservative, and a fragrance.

4. The composition of claim 3, wherein the composition further comprises a solvent, and the solvent is water;
the composition further comprises a humectant, and the humectant is propylene glycol; and the mass ratio of the humectant to the *Crocus sativus* extract is (1400 to 1600): 1, and preferably 1500:1;
the composition further comprises a thickener, and the thickener is hydroxyethyl cellulose; and the mass ratio of the thickener to the *Crocus sativus* extract is (30 to 50): 1, and preferably 40:1;
the composition further comprises a pH adjuster, and the pH adjuster is triethanolamine;
the composition further comprises a solubilizer, and the solubilizer is PEG-40 hydrogenated castor oil; and the mass ratio of the solubilizer to the *Crocus sativus* extract is (4 to 6):1, and preferably 5:1;
the composition further comprises an antioxidant, and the antioxidant is tocopheryl acetate and/or palmitoyl pentapeptide-4; and the mass ratio of the antioxidant to the *Crocus sativus* extract is (0.5 to 1.5): 1, and preferably 1:1;
the composition further comprises a skin conditioner, and the skin conditioner is one or more of dipotassium glycyrrhizinate, *Nymphaea odorata* root extract, hydrolyzed silk and *Camellia sinensis* catechins,; and the mass ratio of the skin conditioner to the *Crocus sativus* extract is (1 to 12):1;
the composition further comprises a preservative, and the preservative is chlorphenesin; and the mass ratio of the preservative to the *Crocus sativus* extract is (15 to 25): 1, and preferably 20:1; and
the composition further comprises a fragrance, and the mass ratio of the fragrance to the *Crocus sativus* extract is (0.5 to 1.5): 1, and preferably 1:1.

5. The composition of claim 4, wherein the skin conditioner is dipotassium glycyrrhizinate, *Nymphaea odorata* root extract and hydrolyzed silk, and the mass ratio of the dipotassium glycyrrhizinate, the *Nymphaea odorata* root extract to the hydrolyzed silk is 10:1:1; or,
the skin conditioner is dipotassium glycyrrhizinate and *Camellia sinensis* catechins, and the mass ratio of the dipotassium glycyrrhizinate to the *Camellia sinensis* catechins is 1:1.

6. The composition of claim 1, wherein the composition comprises the following raw materials in parts by weight: 5 parts of sodium hyaluronate, 1 part of *Crocus sativus* extract, 1 part of *Cladosiphon okamuranus* extract and 1 part of *Chrysanthellum indicum* extract.

7. The composition of claim 1, wherein, the composition comprises, 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Nymphaea odorata* root extract, 0.01% *Cladosiphon okamuranus* extract, 0.01% *Chrysanthellum indicum* extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

8. The composition of claim 1, wherein, the composition comprises, 84.23% water, 15% propylene glycol, 0.4% hydroxyethyl cellulose, 0.05% PEG-40 hydrogenated castor oil, 0.01% palmitoyl pentapeptide-4, 0.05% sodium hyaluronate, 0.01% *Crocus sativus* extract, 0.01% *Camellia sinensis* catechins, 0.01% *Cladosiphon okamuranus* extract, 0.01% *Chrysanthellum indicum* extract, 0.01% dipotassium glycyrrhizinate, 0.2% chlorphenesin and 0.01% fragrance, and the above percentages refer to the mass percentage accounting for the total amount of the raw materials.

9. A preparation method of the composition of any one of claims 1 to 8, wherein the raw material components of the composition are mixed.

10. The preparation method of claim 9, wherein the raw materials contain other component(s) in addition to the sodium hyaluronate, the *Crocus sativus* extract, the *Cladosiphon okamuranus* extract and the *Chrysanthellum indicum* extract, and the other component(s) is/are firstly mixed and then mixed with the sodium hyaluronate, the *Crocus sativus* extract, the *Cladosiphon okamuranus* extract and the *Chrysanthellum indicum* extract.

11. The composition of any one of claims 1 to 8 for use in cosmetics.

12. The composition for the use of claim 11, wherein the cosmetic is a facial mask or an eye mask.

13. The composition for the use of claim 12, wherein the cosmetic is a facial mask, and the raw material components of the facial mask are: a solvent, a humectant, a thickener, a pH adjuster, a solubilizer, an antioxidant, the sodium hyaluronate, the *Crocus sativus* extract, the *Cladosiphon okamuranus* extract, the *Chrysanthellum indicum* extract, *Nymphaea odorata* root extract, hydrolyzed silk, dipotassium glycyrrhizinate, a preservative and a fragrance;
and the preparation method of the facial mask includes the following steps:
(1) mixing a group A and a group B, and homogenizing to obtain a mixture 1; the group A comprises the humectant, and the group B includes the thickener, the pH adjuster, the sodium hyaluronate, the dipotassium glycyrrhizinate, the hydrolyzed silk and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Crocus sativus* extract, the *Cladosiphon okamuranus* extract, the *Chrysanthellum indicum* extract and the *Nymphaea odorata* root extract.

14. The composition for the use of claim 12, wherein the cosmetic is an eye mask, and the raw material components of the eye mask are: a solvent, a humectant, a thickener, a solubilizer, an antioxidant, the sodium hyaluronate, the *Crocus sativus* extract, the *Cladosiphon okamuranus* extract, the *Chrysanthellum indicum* extract, *Camellia sinensis* catechins, dipotassium glycyrrhizinate, a preservative and an fragrance;
and the preparation method of the eye mask includes the following steps:
(1) mixing a group A and a group B, and homogenizing to obtain a mixture 1; the group A includes the humectant, and the group B includes the thickener, the sodium hyaluronate, the dipotassium glycyrrhizinate and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Crocus sativus* extract, the *Cladosiphon okamuranus* extract, the *Chrysanthellum indicum* extract and the *Camellia sinensis* catechins.

15. The composition for the use of claim 13 or 14, wherein the solvent is water;
the humectant is propylene glycol; and the mass ratio of the humectant to the *Crocus sativus* extract is (1400 to 1600): 1, and preferably 1500:1;
the thickener is hydroxyethyl cellulose; and the mass ratio of the thickener to the *Crocus sativus* extract is (30 to 50): 1, and preferably 40:1;
the pH adjuster is triethanolamine;
the solubilizer is PEG-40 hydrogenated castor oil; and the mass ratio of the solubilizer to the *Crocus sativus* extract is (4 to 6):1, and preferably 5:1;
the antioxidant is tocopheryl acetate and/or palmitoyl pentapeptide-4; and the mass ratio of the antioxidant to the *Crocus sativus* extract is (0.5 to 1.5):1, and preferably 1:1;
the preservative is chlorphenesin; and the mass ratio of the preservative to the *Crocus sativus* extract is (15 to 25):1, and preferably 20:1; and
the mass ratio of the fragrance to the *Crocus sativus* extract is (0.5 to 1.5): 1, and preferably 1:1.
